# DEMANDE DE BREVET EUROPEEN

(11) **EP 2 273 039 A1**
(43) Date de publication de la demande: **12.01.2011**
(21) Numéro de dépôt: 10169084.0
(22) Date de dépôt: 09.07.2010
(51) Int. Cl.: E04H 4/12, G01N 33/18

(54) **Contrôle de la qualité de l'eau dans une piscine individuelle**

(30) Priorité: 10.07.2009 FR 0954843
(71) Demandeur: Klereo, 78360 Montesson (FR)
(72) Inventeur: Duchamp, Lionel, 74540, GRUFFY (FR); Saaid, Omar, 78600, MAISON LAFFITTE (FR); Boutet, Jean-Marc, 78230, LE PECQ (FR)
(74) Mandataire: Novaimo

(57) **Abrégé**

Procédé de contrôle de la qualité de l'eau dans une piscine individuelle (1) comprenant des moyens de régulation (3) pilotant des actionneurs (31-34) agissant sur des paramètres physico-chimiques de l'eau et des moyens de supervision (6) bornant l'action des actionneurs (31-34) par des limites de fonctionnement fixées à différents paramètres de fonctionnement, tel qu'il existe au moins deux modes de traitement (M1, M2, M3, M4) dans lesquels au moins certaines limites de fonctionnement sont différentes, un premier mode plus limité étant plus économe en dépense énergétique et/ou en produits qu'un deuxième mode moins limité et tel qu'un passage du premier mode de traitement dans le deuxième mode de traitement est provoqué par les moyens de supervision. Dispositif de contrôle (61,62) pour la mise en oeuvre d'un tel procédé.

## Description

L'invention concerne le domaine de la gestion de la qualité de l'eau dans une piscine individuelle.

Un problème associé à la qualité de l'eau d'une piscine est la possibilité de passage de l'eau dans un état modifié dans lequel l'eau devient trouble (on dit que l'eau a « tourné »), cet état étant qualifié ici d'état dégradé. Un tel état dégradé nécessite une vidange complète et un nouveau remplissage du bassin de la piscine ou nécessite un traitement curatif intense, coûteux et éventuellement nocif pour les utilisateurs ou pour l'environnement du fait de la quantité de produits chimiques à utiliser.

Afin de gérer efficacement la qualité de l'eau d'une piscine, il est courant d'utiliser un dispositif de régulation de la qualité de l'eau. Un tel dispositif comprend plusieurs capteurs et plusieurs actionneurs susceptibles respectivement de mesurer des paramètres physico-chimiques de l'eau, incluant des paramètres bactériologiques, et d'agir sur ces paramètres pour satisfaire un critère de qualité de l'eau.

Par exemple, un dispositif de régulation simple mesure le pH de l'eau et commande un actionneur de type pompe doseuse de manière à obtenir un pH dont la valeur optimale, comprise entre 7.2 et 7.7, dépend des degrés de dureté et d'alcalinité de l'eau et au moins compris dans une plage de tolérance supérieure à 6.9 et inférieure à 7.9.

Plusieurs grandeurs agissent sur la qualité de l'eau et celle-ci est donc définie par un critère de qualité composite, tandis que le dispositif de régulation utilise une technique d'asservissement de type multi-variables, connus de l'homme du métier, pour satisfaire ce critère composite. Toutefois, comme de très nombreux facteurs physico-chimiques ou bactériologiques peuvent être à l'origine de l'apparition d'un état dégradé de l'eau d'une piscine, et que plusieurs types de tels états peuvent se produire, la prévention du passage dans un état dégradé de l'eau d'une piscine individuelle n'est pas toujours efficace avec les solutions existantes.

De plus, les solutions existantes de régulation de la qualité de l'eau de piscines individuelles ne sont pas optimisées et entraînent soit une consommation trop élevée en produits de traitement et/ou en énergie et/ou en eau, ce qui est néfaste pour l'environnement.

L'invention vise à améliorer la gestion de la qualité de l'eau d'une piscine individuelle en introduisant plusieurs modes de traitement avec passage automatique d'un mode de traitement à l'autre, lors de la réception d'une information particulière.

L'invention est particulièrement définie par les revendications.

Notamment, selon l'invention, le procédé de contrôle de la qualité de l'eau d'une piscine individuelle comprenant des moyens de régulation pilotant des actionneurs agissant sur des paramètres physico-chimiques de l'eau pour satisfaire un critère de qualité de l'eau et comprenant des moyens de supervision bornant l'action des actionneurs par des limites de fonctionnement fixées à différents paramètres de fonctionnement, est tel qu'il existe au moins deux modes de traitement dans lesquels au moins certaines limites de fonctionnement sont différentes, un premier mode plus limité étant plus économe en dépense énergétique et/ou en produits de traitement qu'un deuxième mode moins limité, et est tel qu'à la suite de la réception d'une information, les moyens de supervision provoquent automatiquement le passage dans le deuxième mode de traitement bien que le critère de qualité de l'eau soit satisfait dans le premier mode de traitement.
La figure 1 représente une installation de piscine individuelle utilisant le procédé selon l'invention.
La figure 2 représente des moyens de régulation de la qualité de l'eau dans l'installation.
La figure 3 représente schématiquement le procédé selon l'invention, appliqué à quatre modes de traitement de l'eau de la piscine.
La figure 4 représente une première étape du procédé, dans le cas où l'information d'un capteur de présence provoque un premier changement de mode de traitement.
La figure 5 représente une deuxième étape du procédé, dans le cas où l'information d'un serveur météorologique provoque un deuxième changement de mode.
La figure 6 représente schématiquement une zone critique d'un mode de traitement.
La figure 7 représente une troisième étape d'une variante du procédé.

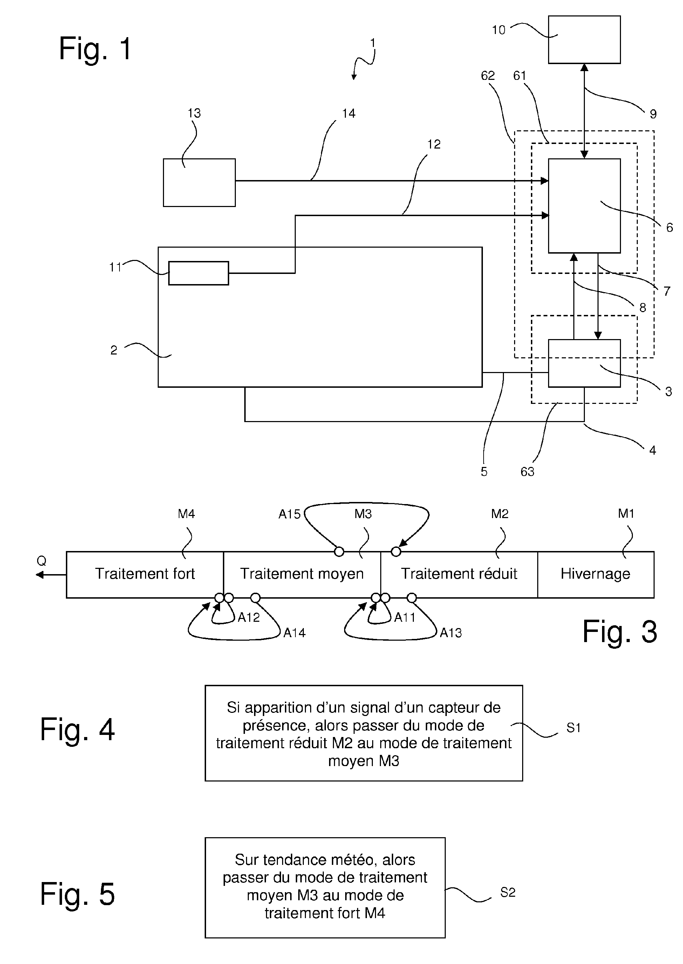

La figure 1 représente une installation 1 de piscine individuelle utilisant le procédé selon l'invention. L'installation comprend un bassin 2 et des moyens de régulation 3 de la qualité de l'eau dans le bassin et raccordés au bassin par un tuyau d'extraction 4 et par un tuyau de refoulement 5.

L'installation comprend également des moyens de supervision 6 communiquant avec les moyens de régulation par un premier moyen de liaison 7, tel qu'une ligne de consigne, et par un deuxième moyen de liaison 8, tel qu'une ligne d'information de régulation. Le premier moyen de liaison et le deuxième moyen de liaison sont avantageusement réalisés par communication radiofréquence bidirectionnelle si les moyens de régulation et les moyens de supervision sont physiquement distants de plusieurs mètres. Une mémoire non représentée peut contenir des données prédéfinies relatives à plusieurs modes de traitement prédéfinis de l'eau, comme cela sera détaillé par la suite.

Les moyens de supervision communiquent avec un serveur distant 10, partagé entre plusieurs installations, par un troisième moyen de liaison 9, tel qu'un réseau Internet. Le serveur distant 10 est dédié à la surveillance d'un parc de piscines individuelles. Alternativement, le serveur distant 10 est un serveur météorologique.

L'installation comprend un capteur de présence 11 immergé dans le bassin et relié aux moyens de supervision. Le capteur de présence est muni de dispositifs optiques ou vibratoires, par exemple ultrasonores, permettant de détecter la présence d'au moins un nageur dans le bassin et de transmettre une information de présence de type logique. La détection de présence peut également être indirecte, par exemple à partir de l'agitation de l'eau du bassin.

Alternativement, les dispositifs optiques ou vibratoires permettent de mesurer ou d'évaluer quantitativement le nombre de nageurs dans le bassin et de transmettre une information de présence de type analogique. L'information de présence est transmise sur une ligne d'information de présence 12, à l'aide de moyens de communication de type filaire, ultrasonore, infrarouge ou radiofréquences.

L'installation comprend également un capteur de sécurité 13 de type périmétrique et relié aux moyens de supervision. Le capteur de sécurité détecte le passage d'un nageur en direction du bassin et transmet une information de sécurité, de type logique, sur une ligne d'information de sécurité 14. Ce capteur peut être constitué par une barrière infrarouge disposée autour du bassin. Alternativement un capteur d'action sur un portillon est utilisé comme capteur de sécurité. L'information de sécurité est transmise aux moyens de supervision à l'aide de moyens de communication de type filaire, ultrasonore, infrarouge ou radiofréquences. Alternativement, le capteur de présence joue également le rôle de capteur de sécurité, ou inversement, afin de permettre notamment la détection de risque de noyade d'un jeune enfant en absence de surveillance adulte.

Un tel capteur de sécurité est en général obligatoire. Son existence est alors avantageusement mise à profit dans le procédé selon l'invention.

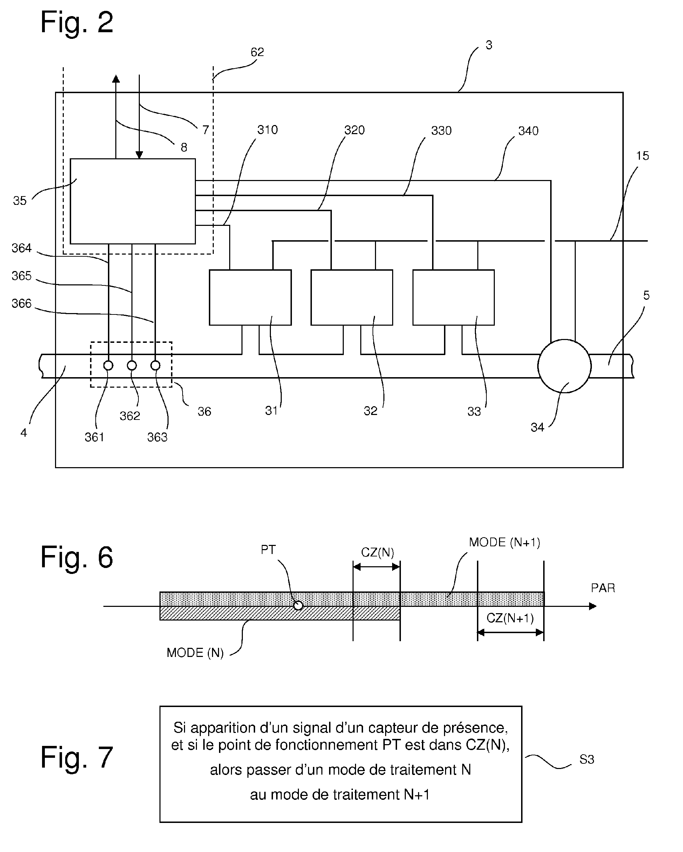

La figure 2 représente des moyens de régulation 3 de la qualité de l'eau dans l'installation 1.

Ces moyens comprennent quatre actionneurs raccordés à un circuit de circulation d'eau allant du tuyau d'extraction au tuyau de refoulement. Un premier actionneur 31 est par exemple une pompe doseuse, permettant l'injection de produits chlorés. Un deuxième actionneur 32 est par exemple un dispositif d'électrolyse. Un troisième actionneur 33 est par exemple un ozonateur. Alternativement, le troisième actionneur est un dispositif à rayonnement ultraviolet. Le quatrième actionneur 34 est un moteur agissant sur une pompe de circulation. L'eau provenant du tuyau d'extraction est ainsi refoulée dans le bassin par le tuyau de refoulement. Ainsi, ces actionneurs permettent l'injection de produits traitants ou la filtration de l'eau de la piscine.

Les actionneurs sont alimentés par une ligne d'alimentation électrique 15, par exemple issue du secteur alternatif, munie de moyens abaisseurs de tension et/ou de protection différentielle non représentés.

Les actionneurs sont commandés par des lignes de commande, respectivement référencées 310-340 pour les actionneurs 31-34. Les lignes de commande proviennent d'un microcontrôleur 35. Un ensemble capteur physico-chimique 36 comprend une première sonde 361, par exemple une sonde de pH, une deuxième sonde 362, par exemple une sonde optique ou une sonde de chlore, et une troisième sonde 363, par exemple une sonde de température de l'eau. D'autres sondes peuvent également être utilisées, par exemple pour la mesure de pression de l'eau ou pour la mesure de concentrations bactériennes. Ces sondes sont raccordées au microcontrôleur par des lignes de mesure, référencées respectivement 364 à 366.

Le microcontrôleur exécute un programme de régulation, de type asservissement multi-variables, apte à satisfaire un critère de qualité de l'eau en pilotant les actionneurs à l'aide des lignes de commande à partir de données récoltées sur les lignes de mesure. Toute technique du domaine de l'automatique peut être utilisée dans le programme de régulation, y compris des règles de système expert ou de logique floue. Dans un cas très simple de régulation mono-variable un asservissement de type PID est utilisé et le critère de qualité est la consigne de l'asservissement PID. Il existe donc une valeur de consigne du critère de qualité, valeur dite aussi d'objectif ou de cible, et une valeur réelle, mesurée ou calculée à partir de mesures obtenues sur les lignes de mesure. Le critère de qualité est satisfait si la valeur réelle diffère très peu de la valeur de consigne, par exemple à moins de 5% ou même à moins de 2%.

Les moyens de supervision et les moyens de régulation sont représentés comme physiquement différents. C'est effectivement le cas si un premier dispositif de contrôle 61 ne comprend que les moyens de supervision, tandis qu'un dispositif de régulation 63 ne comprend que les moyens de régulation.

Alternativement, un deuxième dispositif de contrôle 62 comprend les moyens de supervision mais aussi une partie des moyens de régulation, et notamment le microcontrôleur 35. Une telle disposition permet de regrouper les ressources informatiques matérielles et logicielles nécessaires aux moyens de supervision et de régulation, dans une unité logique et de calcul commune, non représentée.

Pour préserver l'environnement, pour réduire la consommation en produits de traitement ou pour réduire les consommations en énergie ou en eau ou le coût de ces consommations, il est choisi de fixer des limites de fonctionnement à des paramètres de fonctionnement des actionneurs.

Ainsi, on peut décider qu'une pompe doseuse ne peut délivrer plus qu'une certaine quantité de produits chlorés par jour, pour un volume d'eau donné. On peut aussi décider que le moteur de circulation de l'eau doit tourner au plus à la moitié de sa vitesse nominale ou encore qu'il peut tourner uniquement pendant des heures de tarification basse de l'énergie électrique. Ainsi, on entend par limite de fonctionnement tout seuil imposé, qu'un paramètre de fonctionnement d'un actionneur ne peut pas dépasser, qui borne ainsi le fonctionnement de l'actionneur. Une telle limite de fonctionnement peut être temporelle, par la définition de périodes de désactivation ou par un fonctionnement en heures creuses uniquement par exemple, peut être une puissance, par la limitation de la puissance de fonctionnement par exemple, peut être une quantité d'une certaine ressource comme un produit traitant qu'on ne doit pas dépasser. Finalement, une limite de fonctionnement a pour effet de borner la consommation en une ressource de type énergie et/ou en produit traitant et/ou en eau du dispositif de gestion de l'eau de la piscine. Un problème posé par la définition de limites de fonctionnement strictes est que la réduction du traitement par les actionneurs peut compromettre l'atteinte de l'objectif de qualité, rendant même impossible de maintenir un fonctionnement dans la plage de tolérance définie par ces limites de fonctionnement. Le risque est par exemple qu'une pollution soudaine apportée par un groupe de baigneurs, un brassage important de l'eau, un orage ou vent de sable, non traitée du fait des limites de fonctionnement imposées, conduise l'eau dans un état trouble nécessitant par la suite un traitement curatif très important.
Inversement, la définition de limites de fonctionnement trop lâches revient à supprimer l'essentiel des contraintes et à ne pas optimiser les économies de produits ou de consommations.

Selon le concept de l'invention, la gestion de la qualité de l'eau par le dispositif décrit ci-dessus se fait selon plusieurs modes de traitement de l'eau distincts.

Chaque mode se définit notamment par l'ensemble des limites de fonctionnement qui s'appliquent aux actionneurs du dispositif. Les modes sont distincts en ce qu'au moins une de ces limites de fonctionnement est différente entre deux modes différents.

L'invention va maintenant être détaillée avec un exemple de gestion d'une piscine avec quatre modes de traitement prédéfinis. Naturellement, le même concept pourrait être implémenté avec un autre nombre de modes de traitement de l'eau. Toutefois, un nombre limité de modes de traitement sera préférable. Par la suite, les expressions « mode de fonctionnement » et « mode de traitement » sont équivalentes.

La figure 3 représente schématiquement le procédé selon l'invention, appliqué à quatre modes de traitement de l'eau de la piscine. Les modes de traitement sont représentés selon un axe Q orienté selon des quantités croissantes des consommations.

Un premier mode de traitement M1 est un mode d'hivernage, dans lequel les paramètres de fonctionnement des actionneurs sont très largement limités, certains actionneurs pouvant être totalement désactivés.

Un deuxième mode de traitement M2 est un mode de traitement réduit, ou mode d'usage économique, dans lequel la qualité de l'eau est maintenue tout en fixant des limites de fonctionnement importantes, mais globalement moins contraignantes que dans le premier mode de traitement. Ces limites ont été déterminées, par un installateur ou à distance par le serveur 20 s'il est gestionnaire d'un parc de piscines individuelles, en considérant que la piscine n'est pas utilisée ou encore qu'elle est très peu utilisée. Une très faible utilisation correspond par exemple à moins d'un utilisateur pour 40 mètres-cubes de bassin. L'absence d'utilisation ou une très faible utilisation peut aussi être caractérisée par un état de repos total (ou de quasi-repos) de l'eau dans le bassin.

La présence de nageurs joue un grand rôle dans le besoin en produits de traitement : apports bactériens, sudation, brassage de l'eau. II est donc particulièrement avantageux d'anticiper un changement de mode selon une information liée à la présence d'au moins un nageur, et de préférence selon une information du nombre de nageurs, ce nombre étant au moins estimé.

Un troisième mode de traitement M3 est un mode de traitement moyen, ou mode d'usage normal, dans lequel la piscine est normalement utilisée. Une utilisation normale correspond par exemple à une densité d'utilisateurs supérieure au cas précédent mais restant inférieure à un utilisateur pour 10 mètres-cubes de bassin.

Ces densités, données à titre indicatif, peuvent être paramétrées en fonction de la température de l'eau, une eau froide étant plus tolérante à un nombre élevé d'utilisateurs, et/ou en fonction de l'agitation de l'eau provoquée par les utilisateurs. Elles peuvent aussi intégrer des données historiques, et/ou être déterminées par un système expert logé dans le serveur 20.

Les limites de fonctionnement sont moins contraignantes dans le troisième mode de traitement que dans le deuxième mode.

Un quatrième mode de traitement M4 est un mode de traitement fort, ou mode « de choc », dans lequel il convient d'autoriser l'injection de produits de traitement forts. Ce mode de traitement ne comprend pas d'autres limites de fonctionnement que celles imposées par les caractéristiques propres à chaque actionneur et/ou les limites normatives sur les concentrations autorisées de produits.

Ainsi, à chaque mode de traitement correspond l'exécution d'un programme de régulation de la qualité de l'eau. Ce programme de régulation est par exemple identique à des programmes de régulation de l'art antérieur : contrôle de type PID, contrôle par modèle interne, contrôle par logique floue etc.

Selon l'invention, le programme de régulation est différent d'un mode de traitement à l'autre :
- du fait de la présence d'algorithmes différents, d'instructions ou de règles différentes, et/ou
- du fait de valeurs différentes pour certains paramètres et notamment pour des limites de fonctionnement, celles-ci étant prédéfinies dans chaque mode par le constructeur ou par l'installateur ou encore par le propriétaire de la piscine individuelle.

Le procédé est représenté sur la figure 3 sous forme de flèches symbolisant un passage automatique d'un mode de traitement plus économe à un mode de traitement moins économe lorsque les moyens de régulation ne parviennent plus à satisfaire un critère de qualité de l'eau en restant dans les limites de fonctionnement du mode de traitement plus économe. (On dit que le mode de traitement plus économe est saturé).

Ainsi, une première flèche A11 représente un passage automatique, provoqué par les moyens de supervision, du mode de traitement réduit au mode de traitement moyen, si le critère de qualité de l'eau sort d'une plage prédéfinie.

De la même façon, une deuxième flèche A12 représente un passage automatique du traitement moyen au traitement fort si le critère de qualité sort de la plage prédéfinie.

La figure 4 représente une première étape du procédé, dans le cas où l'information d'un capteur de présence provoque un premier changement de mode de traitement.

En effet, l'invention prévoit qu'il est possible de provoquer un passage anticipé d'un mode plus économe à un mode moins économe suite à la réception d'une information par les moyens de supervision, bien que le mode plus économe ne soit pas encore saturé.

Par exemple, dans le mode de traitement réduit, les moyens de régulation n'agissent pas pendant les heures de tarification à tarif élevé. Par contre, si le capteur de présence détecte une agitation de l'eau (ou si le capteur de sécurité détecte l'approche d'un utilisateur) l'information de présence (ou de sécurité) reçue par les moyens de supervision déclenche le passage au mode de traitement normal, avec activation de certains moyens de régulation, bien qu'il soit tout à fait possible que la présence d'un nageur, ou de quelques rares nageurs, ait pu être supportée par l'eau du bassin sans nécessiter d'activer les moyens de régulation. Ce passage anticipé est représenté par une troisième flèche A13.

De la même façon, une quatrième flèche A14 représente un passage anticipé du traitement moyen au traitement fort sur réception d'une information.

Bien que ce cas ne soit pas représenté sur la figure 3, le procédé s'applique de la même façon à un passage du traitement d'hivernage au traitement réduit, que ce soit par anticipation ou lors d'une saturation des moyens de régulation.

La figure 5 représente une deuxième étape du procédé, dans le cas où l'information d'un serveur météorologique transmise aux moyens de supervision provoque un deuxième changement de mode, à savoir un passage du mode de traitement moyen au mode de traitement fort. Cette information est par exemple un risque d'orage important. En effet, un temps orageux provoque facilement un changement d'état de l'eau en favorisant la prolifération bactérienne. Alternativement, l'information est transmise par un serveur de gestion d'un parc de piscines.

Alternativement encore, l'information est fournie par une mini-station météo individuelle, ou simplement par un capteur de température de l'eau (par exemple la troisième sonde 363 peut être utilisée à cet effet). La température de l'eau a en effet une incidence très importante sur sa qualité et les besoins en produits de traitement.

L'avantage de cette anticipation est de prévenir le recours tardif à des phases de traitement beaucoup plus intenses. Un bassin de piscine est en effet un système présentant une inertie considérable et tel que les conséquences sont d'emblée importantes quand elles deviennent mesurables. Il est très avantageux de prévenir les futures modifications de la qualité de l'eau plutôt que d'agir a posteriori. D'où l'intérêt d'utiliser une information sur la présence (et si possible sur le nombre) d'utilisateurs et/ou sur l'agitation de l'eau pour anticiper sur le passage à un mode de traitement plus actif.
Une information précieuse est également celle d'une dérivée négative dans le temps du critère de qualité. Cette dérivée est calculée par les moyens de régulation et transmise aux moyens de supervision par le deuxième moyen de liaison. Alternativement, ce moyen de liaison est utilisé uniquement pour transmettre les valeurs instantanées du critère de qualité, ou de ses composantes, et la dérivée est calculée dans les moyens de supervision.

Le retour dans un mode plus économe est également réalisé de manière automatique, de préférence suite à la réception d'une information. Ainsi, un capteur de présence fonctionnant par détection de l'agitation de l'eau peut fournir une information indiquant que plus aucun nageur ne se trouve dans le bassin. Dans ce cas, le procédé provoque un retour automatique du mode de traitement moyen au mode de traitement réduit, ainsi que représenté par une flèche A15 sur la figure 3. Avantageusement, ce retour automatique a lieu après temporisation fixe, ou liée au nombre de nageurs détecté par le capteur de présence.

La figure 6 représente schématiquement une zone critique CZ d'un mode de traitement, par exemple le mode de traitement réduit M2, pour illustrer l'étape de procédé qui sera décrite en figure 7.

Un paramètre de fonctionnement PAR figure en abscisses et une première zone correspondant à un premier mode de fonctionnement plus économe et désignée par Mode (N) (et hachurée sur la figure) est limitée sur la droite par une première limite de fonctionnement appliquée au paramètre de fonctionnement PAR. Une deuxième zone correspondant à un deuxième mode de fonctionnement moins économe et désignée par Mode (N+1) (et pointillée sur la figure) est limitée sur la droite par une deuxième limite de fonctionnement du paramètre de fonctionnement PAR, supérieure à la première limite. A un instant donné, le paramètre de fonctionnement a une valeur précise, quel que soit le mode de fonctionnement. Cette valeur est représentée par un point de fonctionnement PT.

Le paramètre de fonctionnement est par exemple l'heure et chaque zone correspond alors à une plage horaire autorisée dans chaque mode, ou par exemple une quantité journalière de produits chlorés et chaque zone correspond alors à une plage de quantités journalières autorisées dans chaque mode.

On définit une zone critique CZ(N) à l'intérieure de chaque zone par une relation de proximité avec la limite supérieure du paramètre de fonctionnement. Par exemple, la zone critique correspond à toute valeur supérieure à 90 % de la limite supérieure du paramètre.
On a représenté la zone critique CZ(N) affectée à la première zone et la zone critique CZ(N+1) affectée à la deuxième zone.

La figure 7 représente une troisième étape d'une variante du procédé, en relation avec la figure 6, dans le cas où l'information reçue par les moyens de supervision est une information provenant d'un capteur de présence. Le passage d'un premier mode (N) plus économe, à un deuxième mode (N+1) moins économe, a lieu par anticipation sur réception de cette information (un signal issu du capteur de présence) uniquement si le paramètre de fonctionnement est déjà situé dans la zone critique CZ(N) du premier mode.

Cette variante permet d'éviter un changement automatique de mode de traitement s'il existe une marge encore suffisante dans le mode le plus économe.

De la même façon, la condition sur la zone critique peut être remplacée par une condition temporelle sur le maintien de l'information. Par exemple, le passage anticipé dans le mode moins économe n'a lieu que si la présence est détectée pendant plus de trente minutes.

Ainsi, avec le concept de l'invention, il est possible de connaître l'état de fonctionnement du dispositif de gestion de la qualité de l'eau de la piscine, simplement par la connaissance de son mode de fonctionnement, qui donne une information pertinente sur la situation. Afin de faciliter cette connaissance du mode de fonctionnement, il est possible de prévoir une interface homme machine qui affiche le mode de traitement actif du dispositif, par exemple sous forme de quatre voyants représentatifs des modes M1 à M4 de la description. Le basculement d'un mode de fonctionnement à l'autre peut être aussi réalisé de manière manuelle. De ce fait, le propriétaire de la piscine ou son gestionnaire d'entretien dispose d'une maîtrise complète sur la piscine individuelle. La compréhension du fonctionnement du dispositif et la programmation relative à chaque mode de fonctionnement, notamment en fixant ou en déplaçant des valeurs de limites de fonctionnement, est facile et naturelle. Ainsi, le procédé de contrôle peut se révéler légèrement moins performant que d'autres procédés complexes mis en oeuvre pour des piscines collectives, mais il présente le grand avantage de ne requérir aucune expertise particulière pour sa mise en oeuvre.

## Revendications

1. Procédé de contrôle de la qualité de l'eau d'une piscine individuelle comprenant des moyens de régulation pilotant des actionneurs agissant sur des paramètres physico-chimiques de l'eau pour satisfaire un critère de qualité de l'eau, **caractérisé en ce qu'**il comprend des moyens de supervision bornant l'action des actionneurs par des limites de fonctionnement fixées à différents paramètres de fonctionnement, et tel qu'il existe au moins deux modes de traitement (M1, M2, M3, M4) entre lesquels au moins une limite de fonctionnement est différente, un premier mode plus limité étant plus économe en dépense énergétique et/ou en produits de traitement qu'un deuxième mode moins limité, et **en ce que**, à la suite de la réception d'une information, les moyens de supervision provoquent automatiquement le passage dans le deuxième mode de traitement bien que le critère de qualité de l'eau soit satisfait dans le premier mode de traitement.

2. Procédé de contrôle de la qualité de l'eau selon la revendication 1, **caractérisé en ce qu'**un passage du premier mode de traitement dans le deuxième mode de traitement est provoqué automatiquement par les moyens de supervision quand les moyens de régulation ne parviennent pas à satisfaire le critère de qualité de l'eau dans le premier mode de traitement.

3. Procédé de contrôle de la qualité de l'eau selon la revendication 1 ou 2, **caractérisé en ce qu'**une limite de fonctionnement est temporelle, liée à une puissance ou liée à une quantité d'une ressource.

4. Procédé de contrôle de la qualité de l'eau selon l'une des revendications précédentes, **caractérisé en ce qu'**à chaque mode de traitement correspond l'exécution d'un programme de régulation de la qualité de l'eau.

5. Procédé de contrôle de la qualité de l'eau selon la revendication précédente, **caractérisé en ce que** le programme de régulation est différent d'un mode de traitement à l'autre :
- du fait de la présence d'algorithmes différents, d'instructions ou de règles différentes, et/ou
- du fait de valeurs différentes pour certains paramètres et notamment pour des limites de fonctionnement, celles-ci étant prédéfinies dans chaque mode.

6. Procédé de contrôle de la qualité de l'eau selon l'une des revendications précédentes, **caractérisé en ce que** l'information reçue provient d'un capteur de présence et/ou de sécurité.

7. Procédé de contrôle de la qualité de l'eau selon l'une des revendications 1 à 6, **caractérisé en ce que** l'information reçue est de type météorologique et provient d'un serveur distant.

8. Procédé de contrôle de la qualité de l'eau selon l'une des revendications 1 à 6, **caractérisé en ce que** l'information reçue est une valeur négative de la dérivée par rapport au temps du critère de qualité, provenant des moyens de régulation.

9. Procédé de contrôle de la qualité de l'eau selon l'une des revendications précédentes, **caractérisé en ce que** le passage dans le deuxième mode de traitement n'a lieu que dans une zone, dite critique, dans laquelle au moins un des paramètres de fonctionnement est proche de moins de 10% de sa limite de fonctionnement.

10. Procédé de contrôle de la qualité de l'eau selon l'une au moins des revendications précédentes, **caractérisé en ce qu'**il comprend un mode de traitement réduit dans lequel les limites de fonctionnement ont été fixées pour une eau à l'état de repos dans le bassin.

11. Dispositif de contrôle (61, 62) de la qualité de l'eau dans une piscine individuelle, comprenant au moins un moyen de liaison (9, 10, 12, 14) avec un serveur distant (10) ou avec un capteur de présence et/ou de sécurité (11, 13), **caractérisé en ce qu'**il comprend des moyens de supervision (6) aptes à communiquer avec des moyens de régulation (3) pour exécuter le procédé de contrôle selon l'une au moins des revendications 1 à 10.

12. Dispositif de contrôle (61) selon la revendication précédente, **caractérisé en ce qu'**il comprend des moyens de liaison (7, 8) avec un dispositif de régulation (63) comprenant les moyens de régulation (3).

13. Dispositif de contrôle (62) selon l'une des revendications 11 ou 12, **caractérisé en ce qu'**il comprend au moins un microcontrôleur (35) appartenant aux moyens de régulation (3).

14. Dispositif de contrôle (62) selon l'une des revendications 11 à 13, **caractérisé en ce qu'**il comprend au moins une mémoire apte à contenir des données prédéfinies relatives à plusieurs modes de traitement prédéfini de l'eau.

15. Dispositif de contrôle (62) selon l'une des revendications 11 à 14, **caractérisé en ce qu'**il comprend une interface homme machine qui affiche le mode de traitement actif du dispositif de contrôle.
